# EUROPEAN PATENT SPECIFICATION

(11) **EP 1 970 019 B1**
(45) Date of publication and mention of the grant of the patent: **03.02.2010**
(21) Application number: 08250675.9
(22) Date of filing: 28.02.2008
(51) Int. Cl.: A61B 18/14, A61N 1/05

(54) **A medical use electrical lead including a radio opaque marker**
Elektrischer Leiter mit einem röntgendichten Marker zur medizinischen Verwendung
Fil électrique à usage médical incluant un marqueur radio-opaque

(30) Priority: 12.03.2007 US 906640 P
(43) Date of publication of application: 17.09.2008
(73) Proprietor: Cathrx Ltd, Homebush Bay, NSW 2127 (AU)
(72) Inventor: Anderson, Neil Lawrence, Roseville, NSW 2069 (AU); Chong, Evan, South Strathfield, NSW 2136 (UA)
(74) Representative: Nettleton, John Victor

(56) References cited:
- WO-A-00/28894
- WO-A-2006/012671
- WO-A-2007/014063
- US-A1- 2004 024 397

## Description

### Field

This invention relates, generally, to an electrical lead and, more particularly, to a medical use electrical lead which is desirably used as a component of a catheter.

### Background

The applicant has devised a manufacturing technique for manufacturing a medical use electrical lead, particularly for use as an electrode sheath of a catheter, having an unimpeded lumen. This manufacturing technique embodies the use of a hollow, inner tubular member about which electrical conductors are wound. The electrical conductors are contained between the inner tubular member and an outer jacket on to which electrodes are applied.

A benefit of this manufacturing technique is that the helically wound electrical conductors provide greater flexibility to the electrical lead. In addition, by applying the electrodes on an outer surface of the jacket, greater flexibility of the electrodes themselves results.

Because the applied electrodes are relatively thin, the radio opacity of such electrodes may, at times, be inadequate. It would therefore be beneficial to provide improved radio opacity so that the positions of the electrodes can be more easily determined by a clinician using the medical use electrical lead.

WO 2006/012671 to CathRx Ltd teaches a process for manufacturing an electrical lead having one or more electrodes. The process includes providing an elongate member having at least one polymeric region and further having at least one electrical conductor that extends along at least a part of a length of the elongate member and that is contained in the elongate member. A length of the at least one electrical conductor is accessed at the at least one polymeric region. An electrically conductive adhesive is applied to the length of the at least one electrical conductor that has been accessed.

### Summary

According to a first aspect of the invention, there is provided a medical use electrical lead which includes
an elongate body member comprising an inner tubular member defining a lumen and a cover member covering the inner tubular member;
at least one electrode defined on an outer surface of the cover member;
at least one pair of conductors arranged between the inner tubular member and the cover member, the at least one pair of conductors being in electrical communication with the at least one electrode through the cover member; and
a radio opaque marker circumscribing a periphery of the inner tubular member and underlying the at least one electrode **characterised in that** the radio opaque marker is formed by a winding of an elongate element arranged about the inner tubular member, the winding having a smaller pitch in the region of the at least one electrode.

In this specification, the term "circumscribe" (and derivatives) is to be understood to mean, unless the context clearly indicates otherwise, that the radio opaque marker extends completely about the periphery of the inner tubular member.

Preferably, the radio opaque marker is formed by an elongate element arranged about the tubular member, the elongate element being configured to provide radio-opacity at at least the position of the at least one electrode on the body member. It will be appreciated that, in the case of a multi-electrode lead, each electrode has a radio opaque marker associated with it.

The winding may be a metal wire which is biocompatible and which is sufficiently dense to be radio opaque under x-ray. For example, the wire may be of tantalum, platinum, tungsten, or the like.

The term "smaller pitch" means that the turns of the winding are closer together than the turns of the winding at regions of the body member free of electrodes.

At a distal region of the elongate body member, the winding may have a pitch greater than the pitch of the winding in the region of the at least one electrode to provide kink resistance at the distal region of the elongate body member.

The elongate element may be wound in an opposite sense to the at least one pair of electrical conductors.

The elongate element may underlie the at least one pair of electrical conductors. The elongate element may be covered by a sleeve interposed between the inner tubular member and the cover member. The elongate element may carry an insulating layer.

According to a second aspect of the invention, there is provided a method of forming a medical use electrical lead, the method including
providing an inner tubular member;
arranging at least one pair of electrical conductors helically about the tubular member;
covering the at least one pair of electrical conductors with a cover member;
forming at least one electrode at least partially about the cover member at a predetermined position and placing the electrode in electrical communication with the at least one pair of conductors; and
arranging a radio opaque marker about a periphery of the tubular member to circumscribe a periphery of the tubular member beneath the at least one electrode to provide radio-opacity at the location of the at least one electrode **characterised in that** the radio opaque marker is formed by a winding of an elongate element arranged about the inner tubular member, the winding having a smaller pitch in the region of the at least one electrode.

The method may include arranging the at least one radio opaque marker about the periphery of the tubular member prior to winding the at least one pair of conductors about the tubular member so that the at least one pair of conductors overlies the at least one radio opaque marker.

The method may include winding the elongate element in an opposite sense to the at least one pair of electrical conductors.

The method may include, at a distal region of the inner tubular member, winding the elongate element with a greater pitch than in the region of the at least one electrode to provide kink resistance to the distal region of the inner tubular member.

The method may include interposing a sleeve between the inner tubular member and the cover member and at least partially embedding the elongate element in the sleeve.

According to a third aspect of the invention, there is provided a catheter sheath which includes
an elongate body member comprising an inner tubular member defining a lumen and a cover member covering the inner tubular member;
at least one electrode defined on an outer surface of the cover member;
at least one pair of conductors arranged about the inner tubular member and covered by the cover member and in electrical communication with the at least one electrode through the cover member; and
a radio opaque marker circumscribing a periphery of the inner tubular member and underlying the at least one electrode.

The invention extends also to a catheter which includes a catheter sheath as described above. The catheter is, preferably, a cardio-vascular catheter.

### Brief Description of Drawings

Fig. 1 shows a three dimensional view of a medical use electrical lead in accordance with an embodiment of the invention;
Fig. 2 shows a schematic, sectional side view of a part of the electrical lead; and
Fig. 3 shows a schematic side view of a catheter including the electrical lead functioning as a catheter sheath.

### Detailed Description of Exemplary Embodiment

In the drawings, reference numeral 10 generally designates a medical use electrical lead, in the form of a catheter sheath, or electrode sheath, for a catheter, in accordance with an embodiment of the invention.

The lead 10 comprises a body member 12 having an inner tubular member 14 defining a lumen 16. A plurality of electrical conductors 18 is helically wound about an outer surface of the inner tubular member 14. The electrical conductors 18 are covered by a cover member or jacket 20 of the body member 12. The tubular member 14 and the jacket 20 are of a thermoplastic elastomeric material and, preferably, are of polyester block amide such as that sold under the Registered Trademark, PEBAX^{®}.

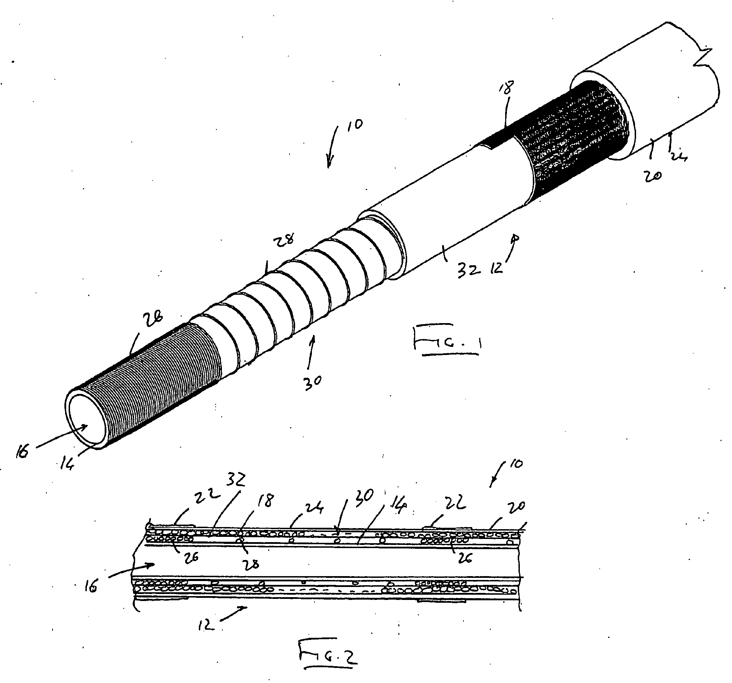

A plurality of electrodes 22 (Fig. 2) is applied about an outer surface 24 of the jacket 20. The electrodes 22 are arranged at longitudinally spaced intervals along the length of the jacket 20. The electrodes 22 are deposited in a suitable manner, for example, by pad printing or other deposition techniques to provide flexible electrodes.

The electrical lead 10 includes a radio opaque marker 26 underlying each electrode 22 and circumscribing a periphery of the inner tubular member 14. More particularly, each radio opaque marker 26 is formed by a winding of an elongate element 28 of radio opaque material. More particularly, the winding 28 is wound helically about the inner tubular member 14. At the region of each electrode 22, the winding 28 is wound with a smaller pitch, i.e. with turns of the winding closer together, to form the radio opaque markers 26. The turns of the winding at each position of the radio opaque markers 26 can, if desired, be in abutting relationship whereas, between the electrodes 22, the turns of the winding 28 have a greater pitch as shown generally at 30 in Figs. 1 and 2 of the drawings.

The winding 28 is arranged beneath the electrical conductors 18. In other words, the electrical conductors 18 are helically wound about the inner tubular member 14over the winding 28. The winding 28 is embedded in, or underlies, a sleeve 32 of a plastics material.

To enable an electrical connection to be made between each electrode 22 and its associated conductors 18, a hole (not shown) is laser cut in the jacket 20 and is filled with a conductive adhesive. To inhibit separation of the conductors 18 when hole filling occurs the conductors 18 are parallel bonded. Each electrode 22 may have at least two parallel bonded conductors 18 (a copper-copper pair) associated with it. However, each electrode 22 may have four conductors 18 associated with it being a copper-copper pair for the supply of energy to the electrode 22 and a copper-constantin pair for a thermocouple. Parallel bonding of the conductors 18 inhibits exposing the underlying winding 28 to the conductive material and inhibits the formation of electrical cross-connections to the underlying winding 28. The provision of the sleeve 32 further reduces the likelihood of a short circuit developing between the conductors 18 and the underlying winding 28.

Preferably, the electrical conductors 18 and the winding 28 are wound in opposite senses. For example, the electrical conductor 18 may be wound with a right hand sense about the inner tubular member 14 with the winding 28 being wound in a left hand sense about the inner tubular member 14or vice versa. With this arrangement, the flexibility of the electrical lead 10 is maintained while enhanced kink resistance of the electrical lead 10 is provided.

The winding 28 is of a biocompatible metal which is sufficiently dense to be radio opaque under X-rays. For example, the winding 28 is of tantalum, platinum, tungsten, or the like. The winding 28 is coated with insulation.

The sleeve 32 also provides a smoother, constant cross-section for the body member 12 of the electrical lead 10 and, as indicated above, serves to inhibit electrical cross-connection to the winding 28. The sleeve 32 is also of PEBAX^{®}. The sleeve 32 may be of a softer grade of PEBAX^{®} than that of the jacket 20 to maintain the flexibility of the electrical lead 10.

As indicated above, the electrical lead 10 functions as an electrode sheath 40 of a catheter 42. The catheter 42 includes a catheter handle 44 having a body 46. A first, steering control member 48 is mounted on an axially displaceable carrier 50 at a distal region of the body 46 with a second, projection control member 52 being carried distally of the steering control member 48 on the carrier 50. The steering control member 48 is fast with the carrier 50 while the projection control member 52 is displaceably arranged relative to the carrier 50. The electrode sheath 40 is carried on, and is fast with, a distal end of the projection control member 52.

A steering shaft (not shown) is received in the lumen 16 of the electrode sheath 40. The steering shaft is secured to the handle 44 via a mounting knob 54 and a slide (not shown) is displaceably arranged relative to the knob 54. The slide is fast with the carrier 50, and, hence, the steering control member 48, so that axial displacement of the steering control member 48 facilitates bending and steering of a distal end 54 of the electrode sheath 40. This facilitates steering of the electrode sheath 40 through the vascular system of a patient and also placement of the electrodes 22 at a site in the patient's body to be treated or for diagnostic purposes.

As described in applicant's co-pending Patent Application No. PCT/AU2006/000266 dated 1 March 2006 published as WO 2006 092 014 and entitled "A catheter handle and a catheter assembly including such a handle", it is beneficial to be able to extend the distal end 54 of the electrode sheath relative to the steering shaft contained in the lumen 16 of the electrode sheath 40. This has benefits, for example, to obtain improved tissue-electrode contact or to facilitate access to difficult to access sites in the patient's body. For this purpose, the projection control member 52 is provided. The distal end 54 is extended relative to the steering shaft by a distance of approximately 2cm at most.

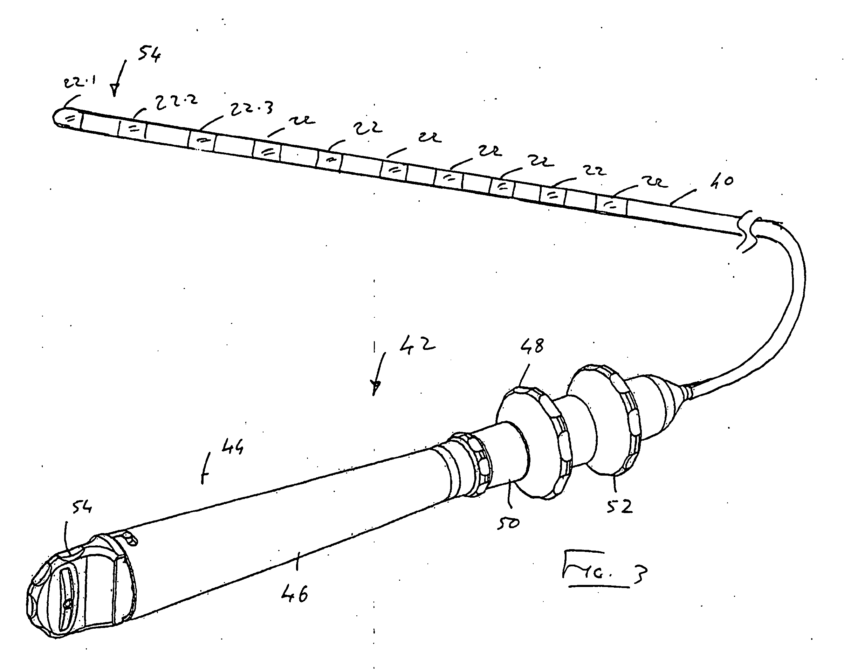

To provide improved kink resistance of the body member 11 of the electrode sheath 40 when extended relative to the steering shaft, the winding 28, proximally of a distal, tip electrode 22.1 (Fig. 3) of the lead 10, is coiled with a slightly narrower pitch than between other electrodes in the sequence of electrodes 22. Thus, more particularly, between a distal, tip electrode 22.1 and its adjacent electrode 22.2, the turns of the winding 28 are closer together than, for example, the turns of the winding 28 between electrodes 22.2 and 22.3. As indicated, this provides improved kink resistance when the distal end 54 of the electrode sheath 40 is in an extended position relative to the steering shaft under the action of the projection control member 52 of the catheter 42.

With the provision of the winding 28 of radio opaque material, when the electrode sheath 40 is inserted into the vasculature of the patient or is at the desired site to be treated, the markers 26 of the winding 28 provide improved radio opacity to enable a clinician to determine the position of the electrodes 22 associated with the relevant markers 26 in the patient's body.

Hence, it is an advantage of the invention that an electrical lead 10 is provided which has improved radio opaque characteristics. These improved radio opaque characteristics are provided in an electrical lead which is not significantly larger, in cross sectional diameter, than an electrical lead without such a radio opaque markers. Hence, the benefit of a more compact electrical lead 10 is still realised.

- It will be appreciated by persons skilled in the art that numerous variations and/or modifications may be made to the invention as shown in the specific embodiments without departing from the scope of the invention as broadly described and defined in the claims. The present embodiments are, therefore, to be considered in all respects as illustrative and not restrictive.

## Claims

1. A medical use electrical lead (10) which includes
an elongate body member (12) comprising an inner tubular member (14) defining a lumen (16) and a cover member (20) covering the inner tubular member (14);
at least one electrode (22) defined on an outer surface of the cover member (20);
at least one pair of conductors (18) arranged between the inner tubular member (14) and the cover member (20), the at least one pair of conductors (18) being in electrical communication with the at least one electrode (22) through the cover member (20); and
a radio opaque marker (26) circumscribing a periphery of the inner tubular member (14) and underlying the at least one electrode (22) **characterised in that** the radio opaque marker (26) is formed by a winding (28) of an elongate element arranged about the inner tubular member (14), the winding (28) having a smaller pitch in the region of the at least one electrode (22).

2. The electrical lead (10) of claim 1 in which, at a distal region of the elongate body member (12), the winding (28) has a pitch greater than the pitch of the winding (28) in the region of the at least one electrode (22) to provide kink resistance at the distal region of the elongate body member (12).

3. The electrical lead (10) of claim 2 in which the elongate element is wound in an opposite sense to the at least one pair of conductors (18).

4. The electrical lead (10) of any one of the preceding claims in which the elongate element underlies the at least one pair of conductors (18).

5. The electrical lead (10) of any one of the preceding claims in which the elongate element is covered by a sleeve (32) interposed between the inner tubular member (14) and the cover member (20).

6. A method of forming a medical use electrical lead (10), the method including
providing an inner tubular member (14);
arranging at least one pair of conductors (18) helically about the tubular member (14);
covering the at least one pair of conductors (18) with a cover member (20);
forming at least one electrode (22) at least partially about the cover member (20) at a predetermined position and placing the electrode (22) in electrical communication with the at least one pair of conductors (18); and
arranging a radio opaque marker (26) about a periphery of the tubular member (14) to circumscribe a periphery of the tubular member (14) beneath the at least one electrode (22) to provide radio-opacity at the location of the at least one electrode (22) **characterised in that** the radio opaque marker (26) is formed by a winding (28) of an elongate element arranged about the inner tubular member (14), the winding (28) having a smaller pitch in the region of the at least one electrode (22).

7. The method of claim 6 which includes arranging the at least one radio opaque marker (26) about the periphery of the tubular member (14) prior to winding the at least one pair of conductors (18) about the tubular member (14) so that the at least one pair of conductors (18) overlies the at least one radio opaque marker (26).

8. The method of claim 6 which includes winding the elongate element in an opposite sense to the at least one pair of electrical conductors (18).

9. The method of any one of claims 6 to 8 which includes, at a distal region of the inner tubular member (14), winding the elongate element with a greater pitch than in the region of the at least one electrode (22) to provide kink resistance to the distal region of the inner tubular member (14).

10. The method of any one of claims 6 to 9 which includes interposing a sleeve (32) between the inner tubular member (14) and the cover member (20) to at least partially cover the elongate element.

## Patentansprüche

1. Elektrische Leitung (10) zur medizinischen Verwendung, die umfasst:
ein längliches Strukturelement (12), das ein inneres rohrförmiges Element (14), das einen Hohlraum (16) definiert, und ein Abdeckelement (20) aufweist, das das innere rohrförmige Element (14) bedeckt;
mindestens eine Elektrode (22), die auf einer äußeren Oberfläche des Abdeckelementes (20) definiert wird;
mindestens ein Paar Leiter (18), das zwischen dem inneren rohrförmigen Element (14) und dem Abdeckelement (20) angeordnet ist, wobei das mindestens eine Paar von Leitern (18) in elektrischer Verbindung mit der mindestens einen Elektrode (22) durch das Abdeckelement (20) ist; und
einen röntgensichtbaren Marker (26), der einen Umfang des inneren rohrförmigen Elementes (14) umschreibt und unter der mindestens einen Elektrode (22) liegt, **dadurch gekennzeichnet, dass** der röntgensichtbare Marker (26) durch eine Wicklung (28) eines länglichen Elementes gebildet wird, das um das innere rohrförmige Element (14) herum angeordnet ist, wobei die Wicklung (28) einen kleineren Wicklungsschritt im Bereich der mindestens einen Elektrode (22) aufweist.

2. Elektrische Leitung (10) nach Anspruch 1, bei der im distalen Bereich des länglichen Strukturelementes (12) die Wicklung (28) einen Wicklungsschritt aufweist, der größer ist als der Wicklungsschritt der Wicklung (28) im Bereich der mindestens einen Elektrode (22), um einen Knickwiderstand im distalen Bereich des länglichen Strukturelementes (12) zu liefern.

3. Elektrische Leitung (10) nach Anspruch 2, bei der das längliche Element in einem entgegengesetzten Richtungssinn zu dem mindestens einen Paar von Leitern (18) gewickelt ist.

4. Elektrische Leitung (10) nach einem der vorhergehenden Ansprüche, bei der das längliche Element unter dem mindestens einen Paar von Leitern (18) liegt.

5. Elektrische Leitung (10) nach einem der vorhergehenden Ansprüche, bei der das längliche Element durch eine Hülse (32) bedeckt wird, die zwischen dem inneren rohrförmigen Element (14) und dem Abdeckelement (20) angeordnet ist.

6. Verfahren zur Herstellung einer elektrischen Leitung (10) zur medizinischen Verwendung, wobei das Verfahren die folgenden Schritte umfasst:
Bereitstellen eines inneren rohrförmigen Elementes (14);
Anordnen des mindestens einen Paares von Leitern (18) spiralförmig um das rohrförmige Element (14);
Bedecken des mindestens einen Paares von Leitern (18) mit einem Abdeckelement (20);
Bilden von mindestens einer Elektrode (22) mindestens teilweise um das Abdeckelement (20) in einer vorgegebenen Position und Anordnen der Elektrode (22) in elektrischer Verbindung mit dem mindestens einen Paar von Leitern (18); und
Anordnen eines röntgensichtbaren Markers (26) um einen Umfang des rohrförmigen Elementes (14) herum, um einen Umfang des rohrförmigen Elementes (14) unterhalb der mindestens einen Elektrode (22) zu umschreiben, um eine Röntgensichtbarkeit an der Stelle der mindestens einen Elektrode (22) zu bewirken, **dadurch gekennzeichnet, dass** der röntgensichtbare Marker (26) durch eine Wicklung (28) eines länglichen Elementes gebildet wird, das um das innere rohrförmige Element (14) herum angeordnet ist, wobei die Wicklung (28) einen kleineren Wicklungsschritt im Bereich der mindestens einen Elektrode (22) aufweist.

7. Verfahren nach Anspruch 6, das den Schritt des Anordnens des mindestens einen röntgensichtbaren Markers (26) um den Umfang des rohrförmigen Elementes (14) herum vor dem Wickeln des mindestens einen Paares von Leitern (18) um das rohrförmige Element (14) herum umfasst, so dass das mindestens eine Paar von Leitern (18) über dem mindestens einen röntgensichtbaren Marker (26) liegt.

8. Verfahren nach Anspruch 6, das den Schritt des Wickeln des länglichen Elementes in einem entgegengesetzten Richtungssinn zu dem mindestens einen Paar von elektrischen Leitern (18) umfasst.

9. Verfahren nach einem der Ansprüche 6 bis 8, das im distalen Bereich des inneren rohrförmigen Elementes (14) den Schritt des Wickelns des länglichen Elementes mit einem größeren Wicklungsschritt als im Bereich der mindestens einen Elektrode (22) umfasst, um einen Knickwiderstand betreffs des distalen Bereiches des inneren rohrförmigen Elementes (14) zu liefern.

10. Verfahren nach einem der Ansprüche 6 bis 9, das den Schritt des Anordnens einer Hülse (32) zwischen dem inneren rohrförmigen Element (14) und dem Abdeckelement (20) umfasst, um mindestens teilweise das längliche Element zu bedecken.

## Revendications

1. Fil électrique à usage médical (10), englobant :
un élément de corps allongé (12), comprenant un élément tubulaire interne (14) définissant une lumière (16), et un élément de couverture (20), recouvrant l'élément tubulaire interne (14) ;
au moins une électrode (22) définie sur une surface externe de l'élément de couverture (20) ;
au moins une paire de conducteurs (18) agencée entre l'élément tubulaire interne (14) et l'élément de couverture (20), la au moins une paire de conducteurs (18) étant en communication électrique avec la au moins une électrode (22) à travers l'élément de couverture (20) ; et
un marqueur radio-opaque (26), entourant une périphérie de l'élément tubulaire interne (14) et agencé au-dessous de la au moins une électrode (22), **caractérisé en ce que** le marqueur radio-opaque (26) est formé par un enroulement (28) d'un élément allongé agencé autour de l'élément tubulaire interne (14), l'enroulement (28) ayant un pas réduit dans la région de la au moins une électrode (22).

2. Fil électrique (10) selon la revendication 1, dans lequel, au niveau d'une région distale de l'élément de corps allongé (12), l'enroulement (28) a un pas supérieur au pas de l'enroulement (28) dans la région de la au moins une électrode (22) pour assurer une résistance à la coudure au niveau de la région distale de l'élément de corps allongé (12).

3. Fil électrique (10) selon la revendication 2, dans lequel l'élément allongé est enroulé dans un sens opposé à la au moins une paire de conducteurs (18).

4. Fil électrique (10) selon l'une quelconque des revendications précédentes, dans lequel l'élément allongé est agencé au-dessous de la au moins une paire de conducteurs.

5. Fil électrique (10) selon l'une quelconque des revendications précédentes, dans lequel l'élément allongé est recouvert par un manchon (32) agencé entre l'élément tubulaire interne (14) et l'élément de couverture (20).

6. Procédé de fabrication d'un fil électrique à usage médical (10), le procédé englobant les étapes ci-dessous :
fourniture d'un élément tubulaire interne (14) ;
mise en place d'au moins une paire de conducteurs (18), enroulée de manière hélicoïdale autour de l'élément tubulaire (14) ;
couverture de la au moins une paire de conducteurs (18) par un élément de couverture (20) ;
formation d'au moins une électrode (22), en moins en partie autour de l'élément de couverture (20), au niveau d'une position prédéterminée, et positionnement de l'électrode (22) en communication électrique avec la au moins une paire de conducteurs (18) ; et
agencement d'un marqueur radio-opaque (26) autour d'une périphérie de l'élément tubulaire (14), pour circonscrire une périphérie de l'élément tubulaire (14) au-dessous de la au moins une électrode (22), afin d'établir une radio-opacité au niveau de l'emplacement de la au moins une électrode (22), **caractérisé en ce que** le marqueur radio-opaque (26) est formé par un enroulement (28) d'un élément allongé agencé autour de l'élément tubulaire interne (14), l'enroulement (28) ayant un pas inférieur dans la région de la au moins une électrode (22).

7. Procédé selon la revendication 6, englobant l'étape d'agencement du au moins un marqueur radio-opaque (26) autour de la périphérie de l'élément tubulaire (14) avant d'enrouler la au moins une paire de conducteurs (18) autour de l'élément tubulaire (14), de sorte que la au moins une paire de conducteurs (18) est superposée audit au moins un marqueur radio-opaque (26).

8. Procédé selon la revendication 6, englobant l'étape d'enroulement de l'élément allongé dans un sens opposé à la au moins une paire de conducteurs électriques (18).

9. Procédé selon l'une quelconque des revendications 6 à 8, englobant, au niveau d'une région distale de l'élément tubulaire interne (14), l'étape d'enroulement de l'élément allongé avec un pas supérieur à celui existant dans la région de la au moins une électrode (22), pour conférer une résistance à la coudure à la région distale de l'élément tubulaire interne (14).

10. Procédé selon l'une quelconque des revendications 6 à 9, englobant l'étape d'agencement d'un manchon (32) entre l'élément tubulaire interne (14) et l'élément de couverture (20) pour recouvrir au moins partiellement l'élément allongé.
